# EUROPEAN PATENT APPLICATION

(11) **EP 3 670 643 A1**
(43) Date of publication of application: **24.06.2020**
(21) Application number: 18382944.9
(22) Date of filing: 19.12.2018
(51) Int. Cl.: C12M 1/12, C12M 1/06

(54) **SUPPORT COIL**

(71) Applicant: Asociación Centro de Investigación Cooperativa en Biomateriales - CIC biomaGUNE, 20014 San Sebastián (ES)
(72) Inventor: Simón Soria, Marcos, 20014 SAN SEBASTIÁN (ES)
(74) Representative: ZBM Patents - Zea, Barlocci & Markvardsen

(57) **Abstract**

A support coil comprises a sheet wound up in a spiral, and a side wall arranged along a longitudinal edge of the sheet, so the side wall remains arranged between successive turns of the coil, and an inner spiral space is defined between the turns and is partly limited by the side wall, wherein the side wall comprises a continuous strip and a discontinuous strip. Also provided are reactors which use such a support coil arranged inside a container such as to be rotatable and cause a flow of liquid along the wound up sheet, from the outside towards the inside of the spiral, as well as methods for producing and operating such reactors for a variety of purposes.

## Description

The present disclosure is related to a support coil and to reactors with a support coil, for example reactors for cell culture, as well as to methods for cell culture and uses of such reactors and support coils.

### BACKGROUND ART

Several kinds of reactors and in particular bioreactors are known, such as bioreactors for cell culture, for example for the culture of adherent cells in the production of therapeutic cells and other biopharmaceuticals. General goals in the design of such reactors are for example providing a reliable culture surface and structures which require a reduced space, allow an efficient flow of culture medium and facilitate the automation of the process.

It has been suggested to provide a culture surface in a bioreactor by employing a membrane wound or rolled up to form a support coil or spiral, arranged to rotate such that the culture medium flows along the turns of the spiral, from the outside towards the inside. This arrangement has been suggested both within rotating and stationary culture chambers. This solution may provide a large cell support surface while requiring a small volume. However, the problems of construction and support of the spiral, as well as effective management of the flow of culture medium, circulation of gas, etc. in the reactor, have not yet been solved efficiently. For example, the culture medium must be retained between successive turns of the spiral to ensure proper feeding of the adhered cells and promote cell culture, but at the same time the available space for the culture medium and process gas decreases in successive turns as the fluid advances towards the centre of the spiral, due to the decrease in diameter, so excess culture medium has to be removed from the spiral. Gas trapped by the culture medium as it advances must also be efficiently removed from the inner spiral space.

Some known reactors use a support coil comprising a spacing layer between the turns, for example a sheet of fibrous material. However, this kind of construction hinders the flow of culture medium throughout the cell culture space and the removal of gas. Other solutions relay on cast, rigid materials set with a shape compatible with the required flow of fluids. However, these solutions require sophisticated industrial processes that preclude the spread of this type of reactors in industrial settings.

The present disclosure aims to address the above problems and provide a reactor that is cost-effective and industrially viable, from the point of view of both manufacture and operation.

### SUMMARY OF THE DISCLOSURE

According to a first aspect of the invention, a support coil is provided. The support coil comprises a sheet wound up in a spiral, and a side wall, whereby an inner spiral space is defined between the turns of the support coil wound up in a spiral and is partly limited by the side wall. The sheet has a length that defines the length of the spiral in the wound up sheet, and a width that defines the width of the support coil. The side wall is arranged along a longitudinal edge of the sheet, at or near a side edge of the support coil, the side wall being arranged between successive turns of the coil. The side wall has a wall length in the direction of the length of the sheet, a wall height in a direction perpendicular to the sheet, and a wall width in the direction of the width of the support coil, and the section of the side wall, in a plane containing the wall length direction and the wall height direction, comprises a substantially continuous longitudinal strip and a discontinuous longitudinal strip.

The provision of the side wall allows manufacturing the coil in a simple and economic way by rolling up a flexible sheet of suitable material, for example a suitable cell culture membrane or substrate, using the side wall as a spacer between the turns of the coil. The side wall may maintain the desired distance between turns, while leaving the inner spiral space free for housing the culture medium and allowing it to flow.

Furthermore, the configuration of the side walls, with a continuous strip or band and a discontinuous strip or band, allows the flow of liquid culture medium, e.g. contained in a partially filled vessel in which the coil may be arranged, along the support coil as it turns about its winding axis when the winding axis is arranged horizontally, and allows the maintenance of a level of culture medium in the inner spiral space between turns. It also allows excess liquid and gas to escape from the inner spiral space, thus providing a uniform fluid flow along the sheet, improving the culture conditions and preventing gas from remaining trapped inside the spiral and hindering the flow of culture medium.

The distance between the turns of the coil is given by the overall height of the side walls, including the continuous and the discontinuous strips.

The support coil may comprise two side walls, for example each arranged along one of the longitudinal edges of the sheet and at or near one side edge of the support coil.

In this case, the two walls may both have the same configuration, as described, or one or the walls may have a different configuration. For example, one side wall may be a solid wall with only a continuous strip, or have strips with a different configuration, or it may have a different height, or other features. In particular, the support coil may have side walls suitable for allowing the escape of liquid and gas from the two sides of the support coil, or only from one side, and suitable for maintaining the desired distance between turns at both sides of the support coil, or only from one side.

The side wall, or each of the side walls, may be provided along a longitudinal edge of the sheet, at or near a side edge of the support coil. The expression "at a side edge" is herein meant to encompass both a case in which the side wall is adjacent the side edge of the coil, i.e. flush with the longitudinal edge of the sheet and side edge of the coil, and also a case in which the side wall is in the vicinity of the side edge, i.e. set back a distance with respect to said edge, for example up to about 10% of the width of the support coil.

The sheet may have a substantially trapezoidal shape, with converging longitudinal side edges, and be wound up forming a spiral with the longest end side on the outside of the spiral and the shortest side at the centre of the spiral.

According to a second aspect, the invention provides a reactor with a support coil as disclosed herein and a container to house the support coil, the container having a bottom to retain liquid; the container may be adapted to retain liquid at a level above the outermost turn of the support coil when the support coil is arranged with a winding axis thereof in a horizontal position. The container may be for example substantially cylindrical.

In such a reactor, the liquid in the container may be easily and efficiently circulated along the spiral, from the outermost turn towards the centre of the spiral, simply by rotating the support coil inside the container, or rotating the container with the support coil inside it.

A third aspect of the invention provides a method for producing a support coil, for example a support coil having one or two side walls configured as a belt. The method may comprise arranging such side wall or walls along one or two longitudinal edges of a sheet, and winding the arrangement to form a coil with the belt or belts arranged between the turns of the coil formed by the wound sheet.

The support coil may comprise a shaft arranged on or along a winding axis of the support coil, i.e. extending in the direction of the axis of the spiral, and attached to the support coil. In some embodiments, a side wall of the support coil may be configured as a toothed belt, and the outer surface of the shaft may be shaped to cooperate with the toothed belt: in this case, the shaft may be rotated to wind up the support coil from the sheet in a method for producing a support coil such as disclosed above.

In a fourth aspect of the invention a method is provided for operating a reactor as disclosed herein, comprising arranging the container such that a winding axis of the support coil is in a horizontal position; providing a liquid in the container to a level above the outermost turn of the support coil; and rotating the support coil about the winding axis. In alternative embodiments, the support coil may be rotated inside the container, or it may be attached to the container and the whole container may be rotated to rotate the support coil.

The method allows providing a continuous and uniform flow of liquid along the wound up sheet.

### BRIEF DESCRIPTION OF THE DRAWINGS

Particular embodiments of the present device will be described in the following by way of non-limiting examples, with reference to the appended drawings, in which:
Figure 1a and 1b are schematic perspective views of two support coils according to examples of the present disclosure, partially unwound;
Figure 2 is a schematic plan view of the sheet forming the support coil of Figure 1a, in unwound condition;
Figure 3a is a section across line AA of Figure 2 and shows the shape of the longitudinal section of one of the side walls of the support coil of Figure 1a;
Figures 3b, 3c and 3d are similar to Figure 3a, showing alternative embodiments of the side wall section;
Figure 4 shows a support coil such as that of Figure 1a, cut in two portions to show the inner structure of a middle part thereof;
Figures 5a and 5b are cross section schematic views of a reactor according to an embodiment of the present disclosure, with a support coil such as that of figure 1a;
Figure 6a and 6b show at an enlarged scale the position of a liquid within a reactor such as that of Figures 5a and 5b, with two different embodiments of the side walls of the support coil;
Figures 7a and 7b are cross section schematic views of a reactor according to another embodiment of the present disclosure, with a support coil such as that of figure 1a;
Figures 8, 9a and 9b schematically illustrate installations with reactors according to embodiments of the present disclosure;
Figure 10a is a plan view of an alternative embodiment of a sheet for a support coil;
Figure 10b is a section along line DD of Fig. 10a;
Figure 10c is a schematic diagram showing the end of a support coil formed with the sheet of Figures 10a, 10b;
Figure 11a is a plan view of another alternative embodiment of a sheet for a support coil;
Figure 11b is a section along line EE of Fig. 11a;
Figure 11c is a schematic diagram showing the end of a support coil formed with the sheet of Figures 11a, 11b;
Figure 12 is a flowchart of embodiments of a method for producing a reactor according to the present disclosure; and
Figure 13 is a flowchart of embodiments of a method for operating a reactor.

### DETAILED DESCRIPTION OF THE INVENTION

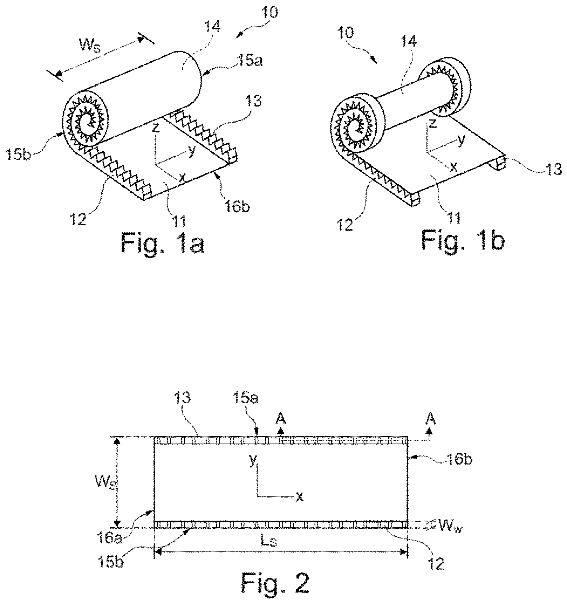

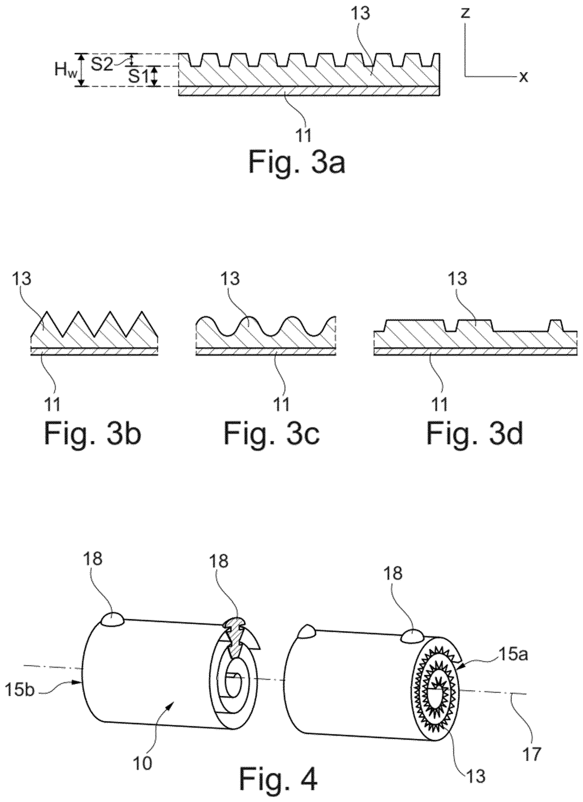

An example support coil 10 for a reactor according to embodiments of the present disclosure is represented in Figures 1a, 1b and 3a-3d, which are merely schematic and not to scale. The proportions in the figures may also be altered, for better clarity. A coordinate system with axes x, y, z has been represented on each figure to facilitate the description and assist understanding.

Figures 1a, 1b show two alternative configurations of the support coil 10, partially unwound. The support coil 10 may comprise a sheet 11 wound up in a spiral, such that an inner spiral space 14 is defined between the turns of the coil 10.

Figure 2 is a plan view of the sheet 11 before being wound up to form the support coil 10, and figure 3a shows a longitudinal cross section of the sheet 11 along lines AAof Figure 2. Figures 3b, 3c and 3d show examples of alternative geometries for the side walls, all comprising a substantially continuous longitudinal strip and a discontinuous longitudinal strip, as will be described below.

Two side walls 12 and 13, which in this example are configured as toothed belts, e.g. timing belts, may be provided along the longitudinal edges 15a, 15b of the sheet 11, for example attached to the sheet itself, for example as shown in Figures 1a (continuous strip adjacent the sheet), or as shown in figure 1b (discontinuous strip adjacent the sheet) such that when the sheet 11 is wound up they remain between successive turns of the coil 10, and at the side edges of the coil: the side walls 12 and 13 may therefore act as spacers between the turns of the coil 10.

Depending on the size, materials and configuration of the sheet, and of the requirements of each particular case, the support coil may be provided with additional spacing elements, for example intermediate walls of any suitable configuration along the length of the sheet, or discrete spacers.

The side walls 12 and 13 also limit at least partly the inner spiral space 14 of the coil 10, at the side edges of the coil. For the purposes of limiting the space as is convenient for the present invention, as will become clear later on, the sheet 11 is wound up such that the discontinuous portion of the side walls is in contact with the convex side of the sheet, as shown in Figures 1a and 1b.

In the present disclosure the following coordinates, terminology and definitions, illustrated in Figures 1a, 1b, 2 and 3a-3d will be used:
- x coordinate: longitudinal or length direction of the sheet 11 and of the side walls 12 and 13 in the unwound sheet and displacement direction of the sheet during the winding of the sheet into a coil and perpendicular to the winding axis.
- **Ls** = length of the sheet 11 between its end edges 16a, 16b. Since the spiral of the coil 10 is formed by the wound up sheet 11, Ls is also the length of the spiral defined by any of the longitudinal edges of the wound up sheet 11 in the support coil 10.
- y coordinate: transverse or width direction of the sheet 11 and the coil 10 and parallel to the winding axis.
   **Ws** = width of the sheet 11 between its longitudinal edges 15a, 15b. Since the longitudinal edges 15a, 15b of the sheet 11 form the side edges of the wound-up coil 10, Ws is also the width of the coil 10 (see Figure 1a).
   **Ww =** width dimension of the side walls 12 and 13.
- z coordinate: height direction of the side walls 12 and 13 perpendicular to the x coordinate and to the y coordinate.
   **Hw** = height of the side walls 12 and 13. Since the side walls 12 and 13 are arranged between turns or the support coil 10 when the side walls define the separation between turns, Hw is also the height of each of the turns of the inner spiral space 14 of the coil 10, i.e. the distance between adjacent turns.

Figure 3a shows a section of the side wall 13 of an unwound support coil along line AA of Figure 2, and therefore in a plane **xz** containing the side wall length direction x and the side wall height direction z. As shown in this figure, the side wall 13 may comprise a substantially continuous longitudinal strip S1, i.e. essentially a strip in which the wall is solid and has no openings, and a discontinuous longitudinal strip S2, i.e. a strip in which the side wall has a plurality of openings or cut out portions of different shapes or geometries.

Figure 3d, for example, shows a side wall having a longitudinal section that is irregular, i.e. where the shape and dimensions of the two strips, and in particular of the discontinuous strip, may vary along the length of the side wall.

In the present disclosure the expression "side wall" is meant to encompass any configuration of an elongate element, with a substantially greater length dimension with respect to its height or width dimensions, for example at least 10 times longer than high and wide, and suitable to be rolled up with a sheet such as sheet 11 of Figures 1a, 1b, 2, 3a-3d, to act as a spacer between the turns of the spiral.

A "strip" of a side wall section is defined herein as an elongated portion or region of the side wall, extending in the length direction thereof. The longitudinal section of a side wall, such as for example the side walls 12 and 13 in Figure 2, may comprise at least two such strips or elongated regions having different configurations, such as S1 and S2 in the examples of Figure 3a-3d.

The expression "substantially continuous" applied to a longitudinal strip according to examples disclosed herein, such as the strip S1 described above, defines a strip that is continuous in most of its length, with no significant openings that would impede maintaining a desired level of fluid in the inner spiral space. For example, a substantially continuous strip will typically be continuous at least along a length encompassing a plurality of openings in the discontinuous strip S2 of the side wall. However, strips comprising for example joints or small gaps are not excluded from the definition of "substantially continuous". For example, side walls may be formed by several elongate sections aligned along the length of the spiral and with their ends attached to each other or simply arranged close to each other, and nevertheless comprise a substantially continuous strip.

Figure 4 shows the support coil 10 cut in two portions, perpendicularly to its winding axis 17, to show the middle part of the coil. As shown, the support coil 10 may comprise at least one fastening means 18 holding together the outermost turn of the coil 10 and at least one of the turns closest to the outermost turn. These fastening means can have different configurations, for example, they may be screws, or pins with a dented stem, or they may also be a welding joint, or adhesive elements, or any other means capable of holding together at least two turns of the coil 10, including the outermost turn. Figure 4 shows three such fastening means, one near each end and one in the middle part, but it may have any suitable number of fastening means.

In embodiments of the present disclosure, a reactor may comprise a support coil according to the present disclosure, for example the support coil 10 of Figures 1a or 1b. In some embodiments, the support coil may be wound around a shaft which is arranged on or along its winding axis 17 and may be attached to the sheet 11, for example through the side walls 12, 13.

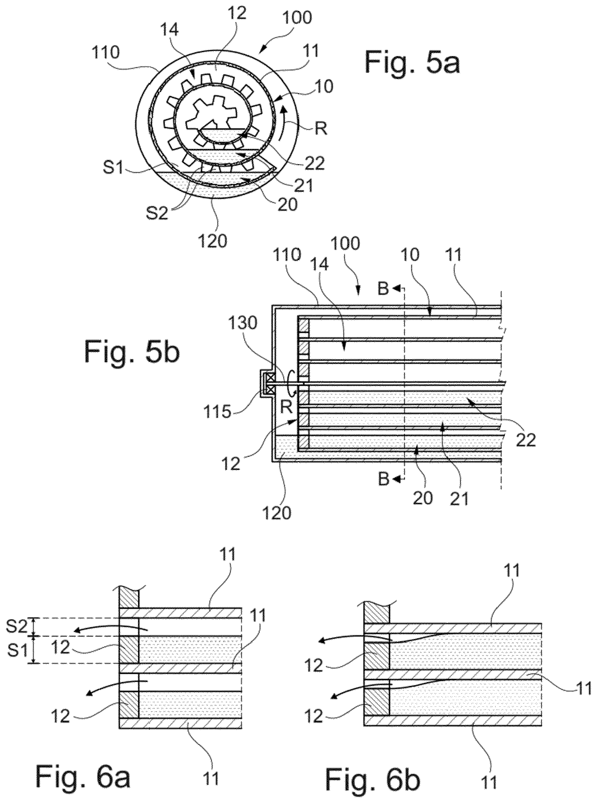

Figures 5a and 5b show schematically some parts of a reactor 100. Figure 5b is a partial longitudinal cross section of the reactor, and Figure 5a is a transverse cross section taken along line BB of Figure 5b. The reactor 100 may be for example a bioreactor for the culture of adherent cells, where cells are adhered to a suitable support and a flow of liquid culture medium is provided to feed the cells.

In Figures 5a, 5b the reactor 100 may comprise a container 110, for example substantially cylindrical, with a reservoir 120 for liquid culture medium at the bottom thereof. In Figure 5a the container may comprise a seat 115 for supporting a shaft 130 in rotation. In use the container 110 is intended to be arranged such that the shaft 130 is in a horizontal position.. A support coil, in the example support coil 10 with side walls 12 and 13 (with only wall 12 being visible in Figures 5a and 5b), is mounted on and around the shaft 130, and the shaft 130 with the support coil 10 may be rotated at a suitable speed inside the container 110, as shown by arrow R.

The skilled person will realize that the shaft may be substituted by other structures with the same purpose, such as a perforated plate on at least one of the side edges of the support coil, the plate having a protuberance at the point where the winding axis crosses the plate and the protuberance being housed in the seat 115 and driven in rotation.

As best visible in Figure 5a, when the support coil 10 rotates in the direction R, liquid culture medium from reservoir 120 will be drawn into the inner spiral space 14 at each rotation of the coil 10, and as the rotation progresses it will proceed along the inner spiral space 14 towards the centre of the spiral.

An amount of culture medium will remain in the lower part of the space between adjacent turns of the coil 10 forming fluid chambers as shown by references 20, 21 and 22, and will progressively be raised towards the centre of the coil 10, such that for example the medium in chamber 20 will be raised to chamber 21 by one rotation of the coil, while at the same time new medium enters in the spiral from the reservoir 120, into chamber 20.

The culture medium is retained in fluid chambers 20, 21, 22 by virtue of the presence of the side wall 12, which partly limits the inner spiral space 14 between turns at the side edges of the coil 10. In particular, the height or level of the medium in each of chambers 20, 21 and 22 is limited by the height of the substantially continuous strip S1 of the side wall 12, as may be seen in the figures, while culture medium that exceeds this level may escape and fall back into reservoir 120 through the openings in discontinuous strip S2, as shown by the arrows in the enlarged detail of Figure 6a. This allows a very convenient management of the culture medium flow, which can flow freely back to the reservoir 120 as the available volume to accommodate the liquid within the spiral space 14 decreases towards the centre of the spiral from fluid chamber 20 to fluid chamber 21, and from here to fluid chamber 22.

The culture medium that reaches the innermost fluid chamber 22 flows then through the openings of the strip S2 near the centre of the spiral, as it is replaced by new culture medium flowing up from fluid chamber 21.

It will be appreciated by those skilled in the art that the flow along the inner spiral space 14, with new liquid being drawn in at each rotation, a level of liquid being maintained between adjacent turns of the coil 10 in fluid chambers 20, 21, 22, and excess liquid automatically flowing out of the spiral, allows an efficient and gentle liquid mixing and distribution system, and wets cyclically and uniformly all the cell culture surface of the support coil 10.

Furthermore, gas pressure that may build up inside the inner spiral space 14 as a consequence of process gas being compressed between the liquid contained in successive fluid chambers may also be released through the openings in the discontinuous strip S2 of the side wall 12, such that it is not trapped in the spiral and does not hinder the flow of culture medium.

As illustrated in Figure 6a, in reactors according to some embodiments the side wall 12 may be configured in such a way that the fluid chambers 20, 21, 22 maintain a level of culture medium that is lower than the height Hw of the side wall 12, so the medium does not reach the convex side of the sheet 11. However, the height of the side walls and the heights of the strips S1 and S2 and the configuration of the discontinuous strip S2, may be designed such that the culture medium fills substantially all the height between turns of the coil in the fluid chambers 20, 21, 22. For example, when the height of the discontinuous strip S2 is sufficiently small, and as shown in the enlarged detail of Figure 6b, capillary forces between the liquid culture medium and the sheet 11 will bring into contact the liquid culture medium and the convex side of the wound up sheet 11.

In this case, both surfaces, the convex one of one turn and the concave one of the next turn farther from the spiral axis, of the sheet 11 may be used for cell adhesion and culture.

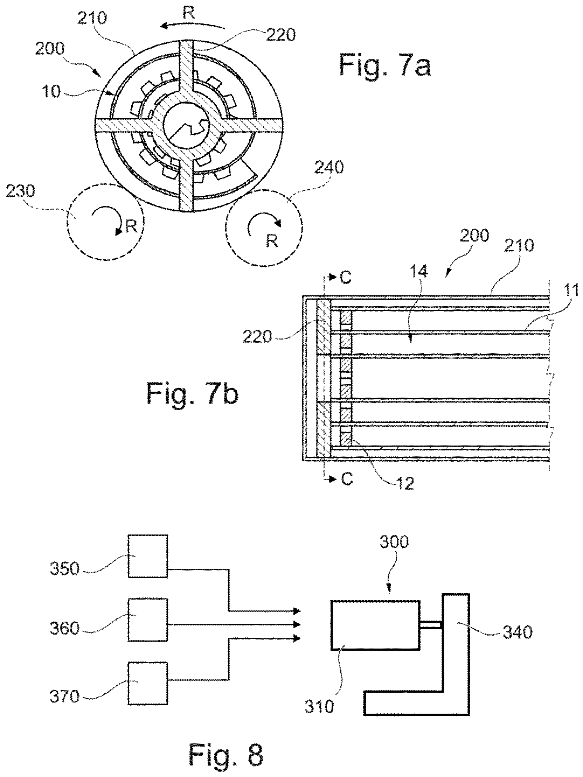

Figures 7a and 7b, which are views similar to those of figures 5a, 5b, where Figure 7a is a transverse cross section taken along line CC of Figure 7b, show a reactor 200 according to another embodiment of the invention, with a container 210 and a support coil 10, but where there is no shaft and the support coil does not rotate with respect to the reactor container. In this embodiment the support coil 10 is firmly attached to a lateral mounting element 220, which in turn is fixed inside the container 210, for example by adhesion, by a mechanical attachment, or simply by pressure fit.

In the embodiment of Figures 7a, 7b, the whole container 210 may be rotated, in order to rotate the support coil 10 and cause the flow of liquid along the spiral. For example, the container 210 may be rotated by being placed on two rotating rollers 230, 240, shown only schematically and in dotted lines in Figure 7a. Arrows R indicate the rotation of the rollers 230, 240 and of the container 210 with the integral support coil 10.

In still another example (not shown), a reactor may be provided with a support coil wound about a shaft, such as in Figures 5a, 5b, but the shaft may be integral with the container, thereby forcing the rotation of the support coil when the container rotates, as in the embodiment of Figures 7a, 7b. For example the shaft 130 of Figures 5a, 5b may be attached to the seat 115 by a keyed joint or other suitable connection to make it integral in rotation with the container.

Figures 5a, 5b and 7a, 7b show particularly simplified examples of reactors. In practice, a reactor may comprise a support coil according to any of the embodiments of the present disclosure, for example a coil having more than 20 or more than 50 turns, with a fluid chamber being formed between each two adjacent turns of the coil. The support coil may comprise at least one side wall having at least one substantially continuous strip which defines the level of fluid in the fluid chamber, and at least one discontinuous strip, with openings for the outflow of culture medium and/or gas. The overall height Hw of the side wall defines the distance between turns.

A reactor according to the present disclosure further comprises other parts and elements, as known to the skilled person, such as for example inlet and outlet ports for culture medium, ports for process gas flow, a harvest port for recovering the product, a heating jacket, ports for probes, etc. The installation for the reactor may further comprise, as also known to the skilled person, culture medium flow controller, pumps, etc., a gas, a motor for rotating the shaft or for otherwise rotating the support coil, a control unit to control the movement of the support coil, processing units to process values of process parameters collected by probes, probes for measuring process parameters such as temperature, pH, CO₂, dissolved oxygen, etc.

For example, Figure 8 shows an example reactor 300 for the culture of adherent cells with a support coil according to embodiments of this disclosure arranged inside a container 310. The reactor 300 further comprises a driving unit 340 for rotating the support coil, a culture medium flow control unit 350, a control unit 360 for the control of process parameters, probes 370, etc.

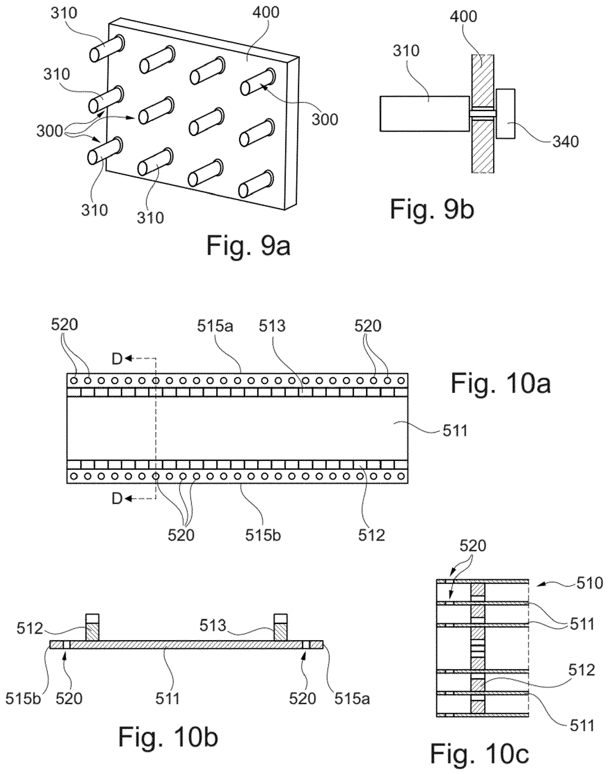

Figure 9a shows a plurality of reactors 300 installed on a wall or panel 400, with the containers 310 of the reactors on one side of the wall and the corresponding driving units 340 on the other side of the wall, as illustrated in Figure 9b, which is a vertical cross section across the wall or panel 400 showing one of the reactors 300 of Figure 9a. For example, the wall or panel 400 may be the wall of a laboratory or controlled area, such that the reactor containers 310 may all be inside a controlled environment, while the driving units 340 and all the control elements for the reactors 300 may be on the other side of the wall 400, outside the laboratory or controlled area.

In embodiments according to the present disclosure, a support coil may have different features from those depicted in the figures.

For example, the support coil may comprise two side walls with a continuous and a discontinuous strip, such as shown above; but it may also have only one such side wall, at one of the side edges of the support coil, and a different kind of side wall at the other side edge, for example a side wall without a discontinuous strip.

In other embodiments the coil may have two side walls with continuous and discontinuous strips, but a side wall may have a configuration different from the other side wall. Furthermore, in some of the embodiments additional intermediate walls may be foreseen, with a similar configuration as those disclosed for the side walls,

In some examples a side wall may have more than one continuous and/or more than one discontinuous strip. For example, a side wall may have a central discontinuous strip between two continuous strips, to facilitate attachment of the side wall to both sides of the sheet during manufacture. Side walls may have the shape of a toothed belt, as above, but also other configurations: for example, the discontinuous strip may have openings of any suitable shape and size.

A side wall may have a uniform overall height Hw and/or uniform strip heights (in the z direction) along all its length, to provide uniform spacing between the turns of the coil, liquid level and liquid outflow section, but may also have variable heights, to adapt to the varying volume of the fluid chambers and/or provide different effects in different regions of the culture medium flow path. Similarly, the geometry of each strip, for example in particular the geometry of the discontinuous strip, may vary along the length of the strip. For example, the geometry or frequency of the openings in the discontinuous strip may vary.

In general, in order to maintain the level of liquid in the fluid chambers, the continuous strip of each side wall may be arranged in contact with the concave side of the wound up sheet. The discontinuous strip may be arranged nearer to the convex side of the wound up sheet than the continuous strip, for example in contact with the convex side if there is only one continuous strip.

The side wall or walls may be discrete elements from the sheet, i.e. manufactured separately and then brought in contact with the sheet, as shown for side walls 12 and 13 above, and may be made of a different material and/or have different properties from those of the sheet. However, they may also be formed as an integral part of a sheet, for example by folding a suitably configured sheet (not shown) along its longitudinal edges. They may also be composite elements, partly formed by the sheet itself and partly attached to it, by adhesion or by any other process.

The side walls may be attached to at least one of the concave side or the convex side of the wound up sheet: for example, the support coil may be wound up with the side walls attached to one side of the sheet, and optionally adhere also to the other side during the winding operation. Alternatively, the side walls may be loose and held in place by the pressure of the sheet once assembled.

The side walls of the sheet, or at least one of the side walls, may be arranged stepped back with respect to the corresponding longitudinal edge of the sheet. This arrangement has the advantage, of making the excess liquid fall first on the same turn of the support coil, where other features such as holes or barriers may channel the fluid flow downwards in a defined and controlled pattern.

For example, Figures 10a, 10b and 10c show an embodiment of a sheet 511 with side walls 512, 513, for example in the shape of toothed belts, that are not arranged flush with the longitudinal edges of the sheet 515a, 515b, but stepped back a distance with respect to these edges. Holes 520 may be provided in the portion of the sheet 511 between each side wall 513, 512 and the corresponding longitudinal edge 515a, 515b.

Figure 10c, which is a cross section showing one end of a support coil 510 formed with the sheet 511, shows that when the sheet is wound up to form the support coil, the side walls 512, 513 will be arranged in the vicinity of the side ends of the support coil. The excess liquid that overflows through the side wall 512 falls on the portion of the sheet with the holes 520, and at least part of it may fall on the underlying turn of the sheet through the holes 520.

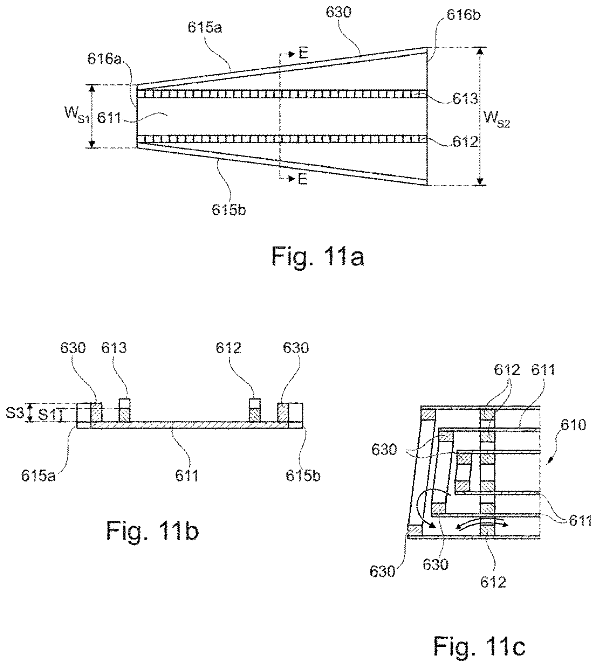

In another embodiment, illustrated in Figures 11a, 11b, 11c, a sheet 611 to be wound up to form a support coil 610 may have a substantially trapezoidal shape, with converging longitudinal side edges 615a and 615b, to be wound up forming a spiral with the shorter end side 616a of the trapeze, having a dimension of Ws₁, at the centre of the spiral, and the longer end side 616b of the trapeze, having a dimension Ws₂, on the outside of the spiral.

Side walls 612 and 613 may be arranged parallel to each other along the longitudinal side edges 615a and 615b. Side walls 612 and 613 may be similar to side walls 12 and 13 of Figures 1a-1d, or may have a different shape and dimension. Additional walls 630 may be arranged parallel to the longitudinal side edges of the sheet and may have different configurations from the side walls 612 and 613, for example they may comprise a continuous strip S3 with a height larger than the height of the continuous strip S1 of side walls 612 and 613, as shown in Figure 11b.

A support coil obtained with the sheet 611 and walls 612, 613, 630 of Figures 11a and 11b has a varying width dimension in the y direction, between WS₁ and Ws₂, and therefore the side ends of the support coil will have a stepped configuration as shown in Figure 11c. Besides, part of the volume of liquid pouring out from one turn of the spiral through the openings in side walls 612 and 613 will fall on the next turn and be forced to partly enter the next fluid chamber farther from the winding axis, thus providing an alternative flow for the liquid culture medium, as shown by the arrows in Figure 11c. This structure is therefore an alternative to the stepped back arrangement of the side wall described for Figures 10a, 10b and 10c, and as the skilled person will realize, both arrangements may be combined to achieve yet other alternative flow patterns for the liquid culture medium.

In embodiments of the invention, the sheet for forming the support coil may be made of any material suitable to be wound up, including for example metals such as aluminum, steel, etc., synthetic polymers such as polyethylene terephthalate (PET), polystyrene, cyclo olefin polymers (COP), polylactic acid, etc., natural polymers such as cellulose, collagen, etc., or others. It may also be made of edible materials, for example in order to produce edible cell rolls. The sheet may also combine areas of different materials.

The surface of the sheet may be treated as convenient for the intended use: for example, it may be chemically modified by the immobilization of enzymes, catalyzers or nanoparticles, ligands or other molecules, or it may be subject to a physical treatment to modify the sheet properties, for example plasma treatment, corona discharge, gamma or beta irradiation, or a mechanical treatment such as engraving the surface with a defined pattern to increase cell adhesion or performance of the sheet during use. Other treatments, or a combination of different treatments, are also possible.

The sheet length and width may range between a few centimeters, for example at least 10 cm, and many meters. A suitable thickness of the sheet depends on the material: it may be for example between 50 and 500 microns for PET, preferably between 100 and 150 microns, with smaller thickness for aluminum or steel. As a general rule, the sheet may be as thin as allowed by the resistance of the material, to reduce its cost and the space it takes up.

The side wall or side walls associated with the sheet, and acting as spacers between the turns of the support coil, may have a width of between 2mm and 10 cm. The choice of the overall height Hw of the side wall or side walls depends on the viscosity of the liquid and on whether the liquid has to wet only one side of the sheet, or both sides of the sheet.

Depending on the application and the scale of the process, the support coil may have very variable width and diameter, ranging from a few centimeters to several meters.

In some embodiments, the support coil may have a width between 5 cm and 300 cm, a diameter between 5 cm and 200 cm, and a distance between turns (i.e. an overall height Hw of the side wall or side walls) between 1 mm and 20 mm, with each of the continuous strip S1 and discontinuous strip S2 having a height of between 0,5 mm and 15 mm.

All the above variants of sheets and support coils, with any suitable combination of materials, sheet shape and dimension, number and configuration of the side walls, etc. may be employed in any of the reactor structures disclosed herein.

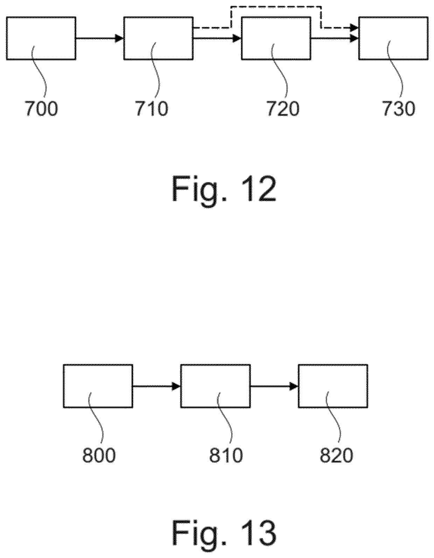

Also provided in the present invention is a method to produce a reactor having a support coil as disclosed herein, comprising, as shown in the flowchart of Figure 12 :
- in block 700, arranging one or two side walls formed as belts, for example as toothed belts, along one or two longitudinal edges of a sheet, and
- in block 710, winding the arrangement formed by the sheet and belt or belts to form a coil in which the belt or belts are arranged between the turns of the coil, such that they act as spacers.

When such a method is used, it may be useful to provide a shaft, such as shaft 130 shown in Figure 5b, and wind the sheet around the shaft. Furthermore, the outer surface of the shaft may be shaped with projections to cooperate with the teeth of the side wall formed as a toothed belt, such that the sheet may be wound up by engaging the teeth or projections of the belt on the shaft and turning the shaft. Other matching or cooperating shapes of the side wall and the shaft may be foreseen. The shaft projections may also be employed to drive the shaft in rotation during use of a reactor.

Optionally, embodiments of the method may also comprise, in block 720 of Figure 12, bonding together at least the two outermost turns of the support coil, such that the coil will remain firmly wound up, for example by using fastening elements 18 shown in Figure 4 or other alternatives. Further turns, e.g. all the turns of the support coil, may be bonded together.

According to embodiments of the present disclosure, in step 730 a support coil such as the support coil obtained in step 710, or bonded as foreseen in step 720, may be mounted within a container, such that when the container is positioned with a winding axis of the coil substantially horizontal, a bottom of the container under the support coil is adapted to retain liquid at a level above the outermost turn of the support coil.

The container and the attachment of the support coil to the container may be any disclosed above.

Also provided in the present disclosure is a method for operating a reactor according to embodiments disclosed herein, the method comprising, as shown in Figure 13:
- in block 800, arranging the container in such a way that the winding axis of the support coil is in a horizontal position;
- in block 810, providing a liquid in the container, to a level above the outermost turn of the support coil; and
- in block 820, rotating the support coil about its winding axis, either by rotating the coil inside the container or by rotating the container with the coil attached inside it, in the direction opposed to the winding direction of the coil.

The rotation of the support coil may cause a continuous and uniform flow of the liquid along the wound up sheet, from the outside towards the inside of the spiral, maintaining a level of liquid in each turn by virtue of the continuous strip of the side wall or walls, and at the same time allowing excess liquid and process gas to be released from each turn through the openings of the discontinuous strip of the side wall or walls.

This may be used for different purposes, such as modifying the composition of the sheet surface by action of reagents contained in the liquid, modifying the composition of the liquid by action of the reagents immobilised on the sheet, or immobilising substances such as nanoparticles, cells or molecules on the sheet.

In some embodiments, the sheet of the support coil may be coated with cells, and the liquid provided in the contained may be cell culture medium.

The method may be a method for the catalytic treatment of a liquid where the wound up sheet has been modified by binding enzymes or other catalyzers that induce a chemical reaction in one or more molecules contained in the liquid flowing through the support coil as it rotates about its winding axis, for example proteins, fatty acids or polysaccharides.

Embodiments of the method may comprise chemical modification of the wound up sheet by contacting the sheet with a liquid containing chemical modifiers, such as chemical reagents, dyes or polymers, that modify the surface of the sheet as the support coil rotates about its winding axis.

In other embodiments the sheet may be coated with a ligand, such as an antibody or other counterpart of an affinity pair, and the liquid may contain the corresponding substrate for the ligand, such as an antigen or the corresponding counterpart of the affinity pair.

Although only a number of particular embodiments and examples have been disclosed herein, it will be understood by those skilled in the art that other alternative embodiments and/or uses of the disclosed innovation and obvious modifications and equivalents thereof are possible. Furthermore, the present disclosure covers all possible combinations of the particular embodiments described. The scope of the present disclosure should not be limited by particular embodiments, but should be determined only by a fair reading of the claims that follow.

## Claims

1. A support coil comprising a sheet wound up in a spiral and a side wall, whereby an inner spiral space is defined between the turns of the sheet wound up in a spiral and is partly limited by the side wall, wherein
• the sheet has a length that defines the length of the spiral in the wound up sheet, and a width that defines the width of the support coil,
• the side wall is arranged along a longitudinal edge of the sheet, at or near a side edge of the support coil, the side wall being arranged between successive turns of the coil,
• the side wall has a wall length in the direction of the length of the sheet, a wall height in a direction perpendicular to the sheet, and a wall width in the direction of the width of the support coil, and
• the section of the side wall, in a plane containing the wall length direction and the wall height direction, comprises a substantially continuous longitudinal strip and a discontinuous longitudinal strip.

2. The support coil according to claim 1 wherein the substantially continuous longitudinal strip is arranged in contact with the concave side of the wound up sheet, and the discontinuous longitudinal strip is arranged between the convex side of the wound up sheet and the substantially continuous longitudinal strip, and optionally in contact with the convex side of the wound up sheet.

3. The support coil according to any one of claims 1 to 2, wherein the side wall is configured as a toothed belt.

4. The support coil according to any one of claims 1 to 3, wherein the side wall is a discrete element from the sheet, attached to at least one side of the wound up sheet.

5. The support coil according to any one of claims 1 to 4, further comprising fastening elements that hold together at least the two outermost turns of the wound up sheet.

6. The support coil according to any one of claims 1 to 5, further comprising a shaft arranged on a winding axis of the support coil and attached to the support coil.

7. A reactor comprising a support coil according to any one of claims 1 to 6 and a container adapted to house the support coil, the container having a bottom to retain liquid, wherein the container is adapted to retain liquid at a level above the outermost turn of the support coil when the support coil is arranged with a winding axis thereof in a horizontal position.

8. The reactor according to claim 7 wherein the support coil and the container are attached to each other forming an integral element.

9. The reactor according to claim 8 wherein the support coil comprises a shaft arranged on a winding axis of the support coil and attached to the support coil, and the container is adapted to rotatably support the shaft.

10. A method to produce a support coil according to any one of claims 1 to 6, wherein the support coil comprises one or two side walls configured as a belt, the method comprising:
• arranging a belt or belts along one or two longitudinal edges of a sheet, and
• winding the arrangement to form a coil with the belt or belts arranged between the turns of the coil formed by the wound sheet.

11. The method of claim 10 further comprising a step of bonding together at least the two outermost turns of the coil.

12. A method to produce a reactor, comprising producing a support coil with the method of claims 10 or 11, and further comprising mounting the support coil within a container such that when the container is positioned with a winding axis of the coil substantially horizontal, a bottom of the container under the support coil is adapted to retain liquid at a level above the outermost turn of the support coil.

13. A method for operating a reactor according to any one of claims 7 to 9, comprising:
• arranging the container such that a winding axis of the support coil is in a horizontal position;
• providing a liquid in the container to a level above the outermost turn of the support coil; and
• rotating the support coil about the winding axis.

14. The method according to claim 13 wherein the liquid is cell culture medium and the sheet is coated with cells.

15. The method according to claim 13 wherein the sheet is coated with catalyst molecules, such as enzymes, and the liquid contains molecules susceptible to be modified by action of the catalyst molecules, such as proteins, fatty acids or polysaccharides.

16. The method according to claim 13 wherein the liquid contains reagents, such as chemical reagents, dyes or polymers, suitable for reacting with the sheet to modify the composition of the surface of the sheet.

17. The method according to claim 13 where the sheet is coated with a ligand, such as an antibody or other counterpart of an affinity pair, and the liquid contains the corresponding substrate for the ligand, such as an antigen or the corresponding counterpart of the affinity pair.
